# EUROPÄISCHE PATENTSCHRIFT

(11) **EP 0 698 051 B1**
(45) Veröffentlichungstag und Bekanntmachung des Hinweises auf die Patenterteilung: **23.07.1997**
(21) Anmeldenummer: 94917630.9
(22) Anmeldetag: 13.05.1994
(51) Int. Cl.: C08G 65/34, C08G 63/672, C08G 18/48, C09J 167/02, C09J 175/08, C09D 167/02, C09D 175/08

(54) **POLYALKYLENGLYKOL**
POLYALKYLENE GLYCOL
POLYALKYLENE GLYCOL

(30) Priorität: 14.05.1993 DE 4316245
(43) Veröffentlichungstag der Anmeldung: 28.02.1996
(73) Patentinhaber: Henkel Kommanditgesellschaft auf Aktien, 40191 Düsseldorf (DE)
(72) Erfinder: FIES, Matthias, D-47800 Krefeld (DE); GRÜTZMACHER, Roland, D-42489 Wülfrath (DE); WESTFECHTEL, Alfred, D-40723 Hilden (DE)
(86) Internationale Anmeldenummer: EP9401553
(87) Internationale Veröffentlichungsnummer: WO9426804

(56) Entgegenhaltungen:
- US-A- 3 188 353

## Beschreibung

Die Erfindung betrifft Polyalkylenglykol, seine Herstellung und Verwendung.

Unter "Polyalkylenglykolen" werden überwiegend lineare Polyether der allgemeinen Formel

H-O-[R-O-]ₙH

verstanden, wobei die Hydroxylgruppen endständig sind und die Alkylen-Gruppe R ein zweiwertiges Radikal ist, das an zwei unterschiedlichen C-Atomen 2 H-Atome weniger enthält als die entsprechende Alkan-Verbindung. Bekannte Polyalkylenglykole sind z.B. Polyethylenglykol (R = CH₂_CH₂), Polypropylenglykol (R = CH₂-CH₂ (CH₃) ) oder Polytetramethylenglykol (R = CH₂CH₂CH₂CH₂). Sie werden in der Regel durch Polyaddition der entsprechenden cyclischen Ether an Wasser oder zweiwertige Alkohole hergestellt. Die bekannten Polyalkylenglykole sind je nach Molmasse flüssige, wachsartige oder feste Produkte. Sie finden vielseitige Anwendung: Vor allem werden sie als Diol-Komponente zur Herstellung von Polyurethanen und Polyestern eingesetzt. Sie bewirken vor allem eine verbesserte hydrolytische Stabilität, verglichen mit den entsprechenden Polyesterdiolen. Außerdem werden sie auch zur Herstellung von nichtionogenen Emulgatoren, Tensiden oder Netzmitteln eingesetzt sowie als Lösungsmittel, Weichmacher oder Lösungsvermittler.

Für ähnliche Zwecke werden auch niedermolekulare Diole eingesetzt, z.B. Ethylenglykol, Hexandiol, 1,10-Decandiol, 1,4-Cyclohexandimethanol oder Dimerdiol.

Das Dimerdiol ist seit langem bekannt. So wurde z. B. vor ca. 30 Jahren in der DE-11 98 348 seine Herstellung durch Dimerisierung von ungesättigten Fettalkoholen mit basischen Erdalkalimetall-Verbindungen bei mehr als 280 °C beschrieben. Sie können auch durch Hydrierung von dimeren Fettsäuren und/oder deren Estern gemäß der deutschen Auslegeschrift DE-B-17 68 313 hergestellt werden. Als Edukte eignen sich hier Dimerisationsprodukte von ein- und/oder mehrfach ungesättigten Fettsäuren und/oder deren Estern, beispielsweise Dimerisationsprodukte von Ölsäure, Linolsäure, Linolensäure, Palmitoleinsäure, Elaidinsäure und/oder Erucasäure und/oder deren Ester. Besonders bevorzugt werden als Edukte Dimerisierungsprodukte von ein- oder mehrfach ungesättigten Fettsäuremischungen wie sie bei der Spaltung von natürlichen Fetten und/oder Ölen, beispielsweise von Olivenöl, Sonnenblumenöl, Sojaöl, Baumwollsaatöl/Korianderöl und/oder Tallöl anfallen. In Abhängigkeit von den gewählten Reaktionsbedingungen der an sich bekannten Dimerisierungen können neben Dimerfettsäuren auch wechselnde Mengen an oligomeren Fettsäuren und/oder Restmengen an monomeren Fettsäuren bzw. deren Estern vorliegen. Enthalten die dimerisierten Fettsäuren bzw. Fettsäureester größere Mengen monomerer Fettsäuren bzw. Fettsäureester, so kann es zweckmäßig sein, diese nach oder vor der Hydrierung zu den Dimerdiolen destillativ abzutrennen, vorzugsweise als Fettsäureester von niederen Alkoholen mit 1 bis 4 C-Atomen. Die Hydrierungen der dimerisierten Fettsäuren bzw. deren Estern können gemäß der deutschen Auslegeschrift DE-B-17 68 313 in Gegenwart von kupfer- und/oder zink-haltigen Katalysatoren in üblichen kontinuierlich arbeitenden Druckhydrierapparaturen mit Gaskreislauf durchgeführt werden. Unter diesen Umständen werden nicht nur die Carboxylgruppen der Fettsäuren zu Hydroxylgruppen hydriert, sondern auch gegebenenfalls noch in den dimerisierten Fettsäuren bzw. deren Estern enthaltene Doppelbindungen zum Teil oder vollständig hydriert. Es ist aber auch möglich die Hydrierung so durchzuführen, daß die Doppelbindungen während der Hydrierung vollständig erhalten bleiben. In diesem Fall fallen ungesättigte Dimerdiole gegebenenfalls in Mischung mit Trimertriolen und Restmonomeren an. Bevorzugt wird die Hydrierung jedoch so durchgeführt, daß die Doppelbindungen zumindest teilweise oder vollständig hydriert werden. Eine weitere Möglichkeit zur Herstellung von vollständig gesättigten Dimerdiolen besteht darin, gesättigte Dimerfettsäuren durch Hydrierung in die entsprechenden gesättigten Dimerdiole zu überführen. Eine andere Möglichkeit zur Herstellung von Dimerdiolen besteht in der Dimerisierung von ungesättigten Alkoholen in Gegenwart von Kieselerde/Tonerde-Katalysatoren und basischer Alkalimetallverbindungen gemäß der internationalen Anmeldung WO 91/13918. Als ungesättigte Alkohole eignen sich ein- und/oder mehrfach ungesättigte Fettalkohole wie Palmitoleyl-, Oleyl-, Elaidyl-, Linolyl-, Linolenyl- und Erucylalkohol. Nach diesem Verfahren enstehen ungesättigte Dimerdiole, deren Doppelbindungen ggf. anschließend teilweise oder vollständig hydriert werden können.

Unabhängig von den beschriebenen Verfahren zur Herstellung der Dimerdiole werden großtechnisch solche Dimerdiole verwendet, die aus Fettsäuren oder deren Estern bzw. Fettalkoholen mit 18 C-Atomen hergestellt worden sind. Auf diese Weise entstehen Dimerdiole mit 36 C-Atomen. Wie bereits geschildert, weisen die Dimerdiole, die nach den oben genannten technischen Verfahren hergestellt worden sind, auch stets wechselnde Mengen an Trimertriolen und Restmonomeren auf. In der Regel liegt dabei der Anteil an Dimerdiolen über 70 Gew.-% und der Rest sind Trimertriole und Monomeralkohole. Es gibt aber auch reinere Dimerdiole mit über 90 Gew.-% Dimerdiolanteil, insbesondere mit über 90 bis 99 Gew.-% Dimerdiolanteil, wobei hiervon solche Dimerdiole wiederum bevorzugt für weitere Reaktionen verwendet werden, deren Doppelbindung zumindest teilweise oder vollständig hydriert ist.

Es ist auch bekannt, das Dimerdiol als Polyol durch Umsetzung mit Diisocyanaten zur Herstellung von Polyurethan-Überzügen zu verwenden. So werden in der DE-12 25 795 Polyurethanlacke aus dimeren und/oder trimeren Fettalkoholen mit einer durchschnittlichen Anzahl von 36 bzw. 54 C-Atomen beschrieben. Vorzugsweise werden die genannten Fettalkohole als alleinige Hydroxylverbindungen verwendet. Es können aber auch geringe Mengen anderer bekannter zur Herstellung von Polyurethanlacken geeigneter Hydroxylverbindungen mitverwendet werden. Die Polyurethanlacke zeichnen sich unter anderem durch eine hohe Beständigkeit gegen hydrolysierende Chemikalien aus, insbesondere gegen Lösungen von alkalisch reagierenden Substanzen. Verglichen wurde dabei mit Polyesterpolyolen.

Weitere Anwendungen zur PU-Herstellung werden in DE 42 15 648 und DE 43 08 100 beschrieben.

Die bekannten Diole zur Herstellung von Polyurethanen und Polyestern sind entweder feste oder hydrophile Verbindungen oder sie haben eine hohe Hydroxylzahl. Das ist in vielen Fällen nachteilig. So ist für die Verarbeitung von Diolen mit einer hohen Hydroxylzahl der hohe Verbrauch an Isocyanat- bzw. an Säure-Komponenten nachteilig. Die Verarbeitung von festen Verbindungen ist oftmals schwierig, z.B. im Hinblick auf die Homogenisierung des Reaktionsgemisches. In vielen Anwendungen beeinflußt die hydrophile Diolkomponente die Produkteigenschaften unerwünscht, z.B. bezüglich ihres Verhaltens gegenüber Wasser (Hydrolysestabilität, Benetzungseigenschaften).

Es bestand daher ein Bedarf nach einem flüssigen hydrophoben Diol mit einer niedrigen Hydroxylzahl.

Die erfindungsgemäße Lösung ist den Patentansprüchen zu entnehmen. Sie besteht in der Bereitstellung eines Polyalkylenglykols der allgemeinen Formel

HO - [ R - O - ]ₙ H

wobei n größer oder gleich 2 und R ein Alkylenrest mit 20 und mehr C-Atomen ist.

Der Alkylen-Rest ist ein zweiwertiges Radikal, das an zwei unterschiedlichen C-Atomen 2 H-Atome weniger enthält als das entsprechende verzweigte, unverzweigte oder cyclische Alkan mit insbesondere 30 und mehr C-Atomen. Vorzugsweise ist er verzweigt und hat mindestens 10 C-Atome zwischen den Radikalstellen. Der Alkylen-Rest leitet sich von primären Alkoholen ab. Die OH-Gruppen der Polyalkylenglykole sind daher endständig und primär. Konkrete Beispiele für die Alkylen-Reste sind die, die sich von folgenden niedermolekularen Diolen ableiten: C₃₆-Dimerdiol, C₄₄-Dimerdiol.

Unter dem Polymerisationsgrad n wird die Anzahl der sich wiederholenden Alkylen-Reste verstanden. Bei molekularuneinheitlichen Produkten ist darunter der durchschnittliche Polymerisationsgrad zu verstehen (DP). Der Polymerisationsgrad von einheitlichen Polyalkylenglykolen ist 2 und größer, insbesondere 4 bis 7. Der durchschnittliche Polymerisationsgrad beträgt mehr als 1,0, insbesondere 1,5 bis 5. Bei den erfindungsgemäßen Polyalkylenglykolen handelt es sich also um Oligomere, deren einzelnen Bestandteile sich z.B. gelchromatographisch trennen lassen. Der Polymerisationsgrad (DP) kann durch die abzuscheidende Wassermenge reguliert werden.

Die erfindungsgemäßen Polyalkylenglykole haben eine Hydroxylzahl (OHZ) von weniger als 175, insbesondere 10 bis 100. Dabei gibt die OH-Zahl an, wieviel mg KOH der Essigsäure-Menge äquivalent sind, die von 1 g Substanz bei der Azetylierung gebunden wird.

Die erfindungsgemäßen Polyalkylenglykole sind bei Raumtemperatur (20 °C) flüssig, d.h. sie haben eine Viskosität bei 25 °C von > 3 000 mPas, insbesondere 3 800 bis 12 000 mPas.

Die erfindungsgemäBen Polyalkylenglykole sind hydrophob, d.h. sie sind bei 20 °C im Wasser praktisch unlöslich, vorzugsweise lösen sich weniger als 1 mg, insbesondere weniger als 0,1 mg in 100 ml Wasser auf.

Die erfindungsgemäßen Polyalkylenglykole können durch Säure-katalysierte Polykondensation von geeigneten niedermolekularen Alkylenglykolen bei erhöhter Temperatur hergestellt werden.
Als Katalysatoren kommen in Frage: Schwefelsäure, Salzsäure, organische Sulfonsäuren (wie Benzol-, Toluol- oder Naphthalinsulfonsäure und Methionsäure = Methandisulfonsäure), ferner Phosphorsäure, Überchlorsäure, Bortrifluorid für sich oder in Verbindung mit einer aromatischen Sulfonsäure sowie saure bzw. leicht hydrolysierbare oder dissozierende Salze (wie Alkalihydrogensulfat, Zinkchlorid, Chinolinhydrochlorid u.a.). Unter diesen spielt die Schwefelsäure sowie die Methansulfonsäure weitaus die wichtigste Rolle.
Die Säure wird in einer Menge von 0,5 bis 30 Gew.-%, insbesondere von 5 bis 15 Gew.-% zugesetzt, bezogen auf das eingesetzte Diol.

Die Kondensationstemperatur beträgt mindestens 150 °C, insbesondere 200 bis 250 °C.

Zur Herstellung der erfindungsgemäßen Polyether wird im allgemeinen die Säure zum Diol hinzugefügt, dann das Reaktionsgemisch auf die Reaktionstemperatur erhitzt, und zwar solange, bis die theoretisch errechnete Menge an Wasser am Wasserabscheider erhalten wurde. Das dauert im allgemeinen 2 bis 20 Stunden, vorzugsweise 6 bis 12 Stunden. Die Ausbeute ist quantitativ. Vorzugsweise wird das Reaktionsprodukt nicht gereinigt. Der Umsatz ist daher vorzugsweise 100 %. Das Reaktionsprodukt ist gelb und klar.

Die erfindungsgemäßen Polyalkylenglykole eignen sich insbesondere als Komponente zur Herstellung von Kunststoffen durch Polykondensation, Polyaddition oder Polymerisation, insbesondere zur Herstellung von Polyestern und Polyurethanen, vor allem von thermoplastischen Polyurethanen mit niedriger Glastemperatur.
Weitere Kunststoffe sind: Polykondensationskunststoffe.

Diese Kunststoffe eignen sich insbesondere zur Verwendung als Basismaterial für Dichtstoffe, Klebstoffe und Beschichtungsstoffe, insbesondere für Lacke. Sie zeichnen sich durch eine hydrophobe Wirkung aus, z.B. durch eine geringe Feuchtigkeitsaufnahme und eine geringe Benetzung mit Wasser. Die mit den erfindungsgemäßen Polyalkylenen hergestellten Kunststoffe sind daher besonders verseifungsstabil, sowohl gegenüber Säuren als auch gegenüber Basen.

Darüber hinaus eignen sich die erfindungsgemäßen Polyalkylenglykole zur Verwendung als Schmieröl, Weichmacher, Textilhilfsmittel.

Die Erfindung wird nun im einzelnen beschrieben.

### Beispiele

### Beispiel 1:

### Polyalkylenglykol mit einer OHZ von 28

678 g (1,3 Mol) Dimerdiol (Sovermol POL 900, OHZ: 208, VZ: 1,8, IZ: 42, SZ: 0,2) und 6,8 g Methansulfonsäure wurden am Wasserabscheider 10 Stunden auf 200 °C erhitzt. Das Endprodukt ist ein gelbes, klares Polyol. Die Ausbeute ist quantitativ. Viskosität = 11900 mPas (Brookfield, 25 °C). OHZ: 28, SZ: 1,8, IZ: 37. GPC: 10 % Polymerisationsgrad 4, 10 % Polymerisationsgrad 5, 20 % Polymerisationsgrad 6, 40 % Polymerisationsgrad 7.

### Beispiel 2:

### Polyalkylenglykol mit einer OHZ von 39

1 000 g (1,9 Mol) Dimerdiol (Sovermol POL 900, OHZ: 208, VZ: 1,8, IZ: 42, SZ: 0,2) und 10,0 g Methansulfonsäure wurden am Wasserabscheider 8 Stunden auf 200 °C erhitzt. Das Endprodukt ist ein gelbes, klares Polyol. Die Ausbeute ist quantitativ. OHZ: 39, SZ: 2,7.

### Beispiel 3:

### Polyalkylenglykol mit einer OHZ von 15,5

1 000 g (3,7 Mol) Dimerdiol (Sovermol POL 900, OHZ: 208, VZ: 1,8, IZ: 42, SZ: 0,2) und 10,0 g Methansulfonsäure wurden am Wasserabscheider 8 Stunden auf 240 °C erhitzt. Das Endprodukt ist ein gelbes, klares Polyol. Die Ausbeute ist quantitativ. OHZ: 15,5, SZ: 1,5. GPC: 7 % Polymerisationsgrad 4, 7 % Polymerisationsgrad 5, 25 % Polymerisationsgrad 6, 45 % Polymerisationsgrad 7.

Die Abkürzungen haben folgende Bedeutung:
OHZ = Hydroxylzahl.
Sie gibt an, wieviel mg Kaliumhydroxid der Essigsäure-Menge äquivalent sind, die von 1 g Substanz bei der Acetylierung gebunden wird. Die Probe wird mit Essigsäureanhydrid-pyridin gekocht und die entstehende Säure mit KOH-Lösung filtriert.

VZ = Verseifungszahl.
Kenngröße für die in dem Diol enthaltene Konzentration an Estern.

JZ = Jodzahl.
Dabei handelt es sich um eine Maßzahl für den Grad der Ungesättigtheit der Diole. Die im Molekül vorhandenen Doppelbindungen werden mit elementarem Brom titriert und der Verbrauch auf Jod umgerechnet.

SZ = Säurezahl.
Dabei handelt es sich um eine Maßzahl für den Gehalt an freien organischen Säuren in dem Diol. Sie gibt die Anzahl der mg an KOH an, die zur Neutraliation von 1 g der Diole verbraucht werden.

GPC = Gel-Permeations-Chromatographie.
Dabei werden die Oligomere auf folgende Weise voneinander getrennt: PL-Gel-System: PL-Gel Vorsäule, 2 x 100 A, 2 x 50 A,
EM: THF 1 ml/min, 40 °C.

Viskosität: nach Brookfield bei 25 °C, Model DV-II, Spindel 21.

## Patentansprüche

1. Bei 20 °C flüssige Polyalkylenglykole, gekennzeichnet durch einen Alkylen-Rest mit 20 oder mehr C-Atomen und einem Polymerisationsgrad von 2 oder mehr, mit der Maßgabe, daß
a) die OH-Gruppen der Polyalkylenglykole endständig und primär sind und
b) die Polyalkylenglykole eine Hydroxylzahl von weniger als 175 aufweisen.

2. Polyalkylenglykole nach Anspruch 1, gekennzeichnet durch einen verzweigten Alkylen-Rest mit mindestens 10 C-Atomen in der Hauptkette.

3. Polyalkylenglykole nach Anspruch 1, gekennzeichnet durch einen Polymerisationsgrad von 4 bis 7.

4. Herstellung der Polyalkylenglykole nach den Ansprüchen 1, 2 oder 3 durch Säure-katalysierte Polykondensation aus monomeren Alkylenglykolen bei hohen Temperaturen.

5. Verwendung der Polyalkylenglykole nach den Ansprüchen 1, 2 oder 3 zur Herstellung von Polyestern und Polyurethanen, insbesondere thermoplastischen Polyurethanen.

6. Verwendung der Polyurethane und der Polyester nach Anspruch 5 zum Kleben, Dichten und Beschichten, insbesondere zum Lackieren.

## Claims

1. Polyalkyleneglycols, which are liquid at 20 ° C, characterized by an alkylene radical containing 20 carbon atoms or more and having a aegree of polymerization of 2 or more and with the condition that
a) the OH-functions of the poly alkylene glycols are terminal and primary and
b) the polyalkylene glycols have a hydroxyl value of less than 175.

2. Polyalkylene glycols as claimed in claim 1, characterized by a branched alkylene radical containing at least 10 carbon atoms in the main chain.

3. Polyalkylene glycols as claimed in claim 1, characterized by a degree of polymerization of 4 to 7.

4. A process for the production of the polyalkylene glycols claimed in claim 1, 2 or 3 by acid-catalyzed polycondensation of monomeric alkylene glycols at high temperatures.

5. The use of the polyalkylene glycols claimed in claim 1, 2 or 3 for the production of polyesters and polyurethanes, more particularly thermoplastic polyurethanes.

6. The use of the polyurethanes and polyesters as claimed in claim 5 for bonding, sealing and coating, more particularly for painting.

## Revendications

1. Polyalkylène glycols liquides à 20°C,
caractérisés par
un radical alkylène de 20 ou plus d'atomes de carbones et un degré de polymérisation de 2 ou plus avec la condition, que
a) les groupes OH des polyalkylène glycols soient terminaux et primaires et
b) les polyalkylène glycols possèdent un indice hydroxyle inférieur à 175.

2. Polyalkylène glycols selon la revendication 1,
caractérisés par
un radical alkylène ramifié comportant au moins 10 atomes de carbone dans la chaîne principale.

3. Polyalkylène glycols selon la revendication 1,
caractérisés par
un degré de polymérisation de 4 à 7.

4. Fabrication de polyalkylène glycols selon les revendications 1, 2 ou 3 par polycondensation à des températures élevées, catalysée par des acides, à partir d'alkylène glycols monomères.

5. Utilisation de polyalkylène glycols selon les revendications 1,2 ou 3 pour la fabrication de polyesters et de polyuréthannes, en particulier de polyuréthannes.

6. Utilisation des polyuréthannes et des polyesters selon la revendication 5 pour le collage, l'étanchéisation et l'enduction, en particulier pour le vernissage.
